# EUROPEAN PATENT APPLICATION

(11) **EP 4 454 448 A1**
(43) Date of publication of application: **30.10.2024**
(21) Application number: 24171622.4
(22) Date of filing: 22.04.2024
(51) Int. Cl.: A01C 3/06, G01N 21/359, G01N 33/24, A01C 21/00

(54) **MANURE SPREADER FOR THE PRECISE APPLICATION**

(30) Priority: 27.04.2023 PL 44463123
(71) Applicant: Uniwersytet Przyrodniczy w Poznaniu, 60-637 Poznan (PL)
(72) Inventor: Czechlowski, Miroslaw, 61-616 Poznan (PL); Wojciechowski, Tomasz, 62-081 Przezmierowo (PL); Prawniczak, Stanislaw, 62-200 Gniezno (PL)
(74) Representative: Kuczynska, Teresa

(57) **Abstract**

A manure spreader for the precise application of solid organic fertilisers, comprising a near-infrared spectrometer (2) together with a measuring probe installed in the loading box (3) of the manure spreader equipped with a floor conveyor (6) and a spreading adapter (1) in which the measuring probe of the near-infrared spectrometer (2) is mounted in a holder (11) of the spectrometer probe (2) on a mounting plate (9) installed on the side wall of the manure spreader loading box (3) in that part of the manure spreader loading box (3), which is directly adjacent to the spreading adapter (6) and is spaced from it not more than 100 cm and is positioned in the lower half of the manure spreader loading box (3) above the floor conveyor (6) but not more than 30 cm above the floor conveyor (6), directly in front of the measuring probe of the near-infrared spectrometer (2) there is a set of retaining tines (12) set coaxially on a rotary axis (13) connected to a lever (15) driven by a hydraulic cylinder (14).

## Description

The object of the invention is a device for the precise application of solid organic fertilizers especially in manure spreaders and a method for the precise application of solid organic fertilizers enabling application especially in manure spreaders which are commonly used in agriculture. The device and method allow the evaluation of the NKP macronutrient content, expressed in kg/t or kg/m3, in basic solid organic fertilisers directly under field conditions during the operation of a spreader designed for these fertilisers.

Various fertiliser spreaders, including those for organic fertiliser are known from the prior art. Some of them have various types of sensors that, based on the prior mapping of the field, allow the right amount of fertiliser to be dosed at a given point in the field, based on the predetermined vegetative demand of a particular section of the field.

Field mapping systems based on vegetation coefficients are also known to assess the diversity of a field, if only in terms of its different NPK macronutrient requirements. This allows the right amount of fertiliser to be tailored to each zone of the field, resulting in optimum vegetation.

Various data sources are used for field mapping, e.g., soil sample analyses, yield mapping, aerial or satellite remote sensing, spectrometric sensors and hyperspectral cameras mounted on agricultural machinery, etc. Maps of vegetation indices drawn up in this way, or the resulting direct maps of variable fertiliser rates, make it possible to determine precisely what the optimum fertiliser rate is for individual macronutrients at a given point in the field.

There are also known seeders and spreaders for mineral fertilizers and spreaders for liquid organic fertilizers, which apply the optimal dose of artificial or natural fertilizer based on the mapping of vegetation indices. Both mineral and natural liquid fertilisers have a defined, or readily ascertainable, fixed chemical composition as well as a resulting specific amount of NPK macronutrients expressed in kg of pure element per tonne of fertiliser. During their field application, they are therefore not required to undergo additional real-time verification for possible changes in fertiliser composition.

However, the precise application of solid organic fertilisers, based on the same variable rate fertiliser maps, is problematic. Due to the variable and not always known composition of solid organic fertilisers, additional real-time monitoring is needed during spreader operation. Such systems that monitor solid organic fertilisers using optical probes are also known from the prior art. However, they are characterised by a low degree of test accuracy.

A major problem affecting the accuracy of measurements carried out in this way is the high compactness of the manure entering the spreader loading box. As a result, there are large voids between the fertiliser 'clumps' that periodically prevent measurements with optical probes requiring direct contact with the material to be analysed.

The aim of the invention is to solve the problem that there is a lack of measuring systems and machines equipped with them on the market to measure the organic solid fertilisers' NPK macronutrients abundance, in real time, directly during the application of these fertilisers by means of a manure spreader on the field surface. The use of such measuring systems in manure spreaders will enable NPK macronutrients to be dosed in a way that is adapted to the predetermined vegetation demand of the field. This will enable the integration of organic fertiliser application into precision farming technology.

The proposed device allows the solid organic fertilisers' NPK macronutrients abundance to be measured using a near-infrared spectrometer installed in a manure spreader, and the results obtained in this way can become the basis for optimising the application of this type of fertiliser in the field, based on previously drawn up maps of variable fertiliser doses.

The essence of the invention is the use of a near-infrared spectrometer in a manure spreader, which, using the diffuse reflection method, records infrared radiation absorbance spectra for successive manure samples moving in front of the spectrometer probe as a result of the operation of the floor conveyor. A suitable calibration model, selected according to the type of fertiliser to be applied (cattle manure, chicken manure, compost, etc.) then converts the acquired absorbance spectrum into NPK macronutrient abundance. On this basis, the spreader controller, via a standard proportional valve, controls the displacement speed of the floor conveyor to match the NPK macronutrient dosage to the dosage chosen by the user, expressed in kg of pure element per hectare, or to the application map used to allow variable fertiliser dosages.

The device for the precise application of solid organic fertilisers especially in manure spreaders consists of a near-infrared spectrometer with a measuring probe installed in the loading box of a manure spreader equipped with a floor conveyor and spreading adapter. The measuring probe of the near-infrared spectrometer is mounted in the probe holder of the spectrometer on a mounting plate installed on the side wall of the loading box of the manure spreader in that part of the loading box of the manure spreader which is directly adjacent to the spreading adapter, and is positioned in the lower half of the manure spreader loading box above the floor conveyor, while immediately in front of the measuring probe there is a set of organic manure sample retaining tines set coaxially on a rotating axis connected to a lever set in motion by a hydraulic cylinder.

The mounting plate is installed on the side wall of the manure spreader loading box and the measuring probe installed on it is in a favourable position, i.e. no more than one metre away from the spreading adapter. It is advantageous if the measuring probe on the mounting plate is installed at a height of no more than 300 mm above the floor conveyor and no less than 100 mm above the floor conveyor.

It is advantageous if there is an inspection window on the side wall of the manure spreader loading box and the mounting plate where the spectrometer probe holder is mounted.

The method for the precise application of solid organic fertilisers especially in manure spreaders consists of the steps of: mapping the vegetation of the field, placing the solid organic fertiliser in the loading box of the manure spreader, testing the composition of the organic fertiliser using the measuring probe of a near-infrared spectrometer, sending the results via an interface (USB, Ethernet, CAN Bus) to the controller managing the operation of the manure spreader, adjusting the application rate of the fertiliser spread by the adapter to the results obtained estimating the fertiliser's NPK macronutrient abundance using the spectrometer by varying the speed of the floor conveyor, whereby, prior to analysing the composition of the organic fertiliser using the measuring probe of the near-infrared spectrometer, the extending piston rod of the hydraulic cylinder causes the fertiliser sample retaining tines to slide inside the loading box of the manure spreader and simultaneously to move them to the front of the box, there is a counter-direction of this movement in relation to the movement of the fertiliser inside the loading box and the fertiliser is compressed and pressed against the loading box wall where the measuring probe is located and the composition of the fertiliser is estimated with the spectrometer on the basis of the absorbance spectrum acquired with the measuring probe, once the measurement has been taken, the piston rod of the cylinder retracts into the cylinder releasing the retained sample of fertiliser, the retaining teeth retract into the wall of the loading box allowing further free flow of material until the next measurement begins.

The main advantage of the device is the ability to evaluate the abundance of fertiliser directly during its application in the field, and the results of the real-time measurements can be transmitted via common interfaces (USB, Ethernet, CAN-Bus) to the controller managing the operation of the manure spreader. Based on this, the fertiliser application control algorithm can control the displacement speed of the spreader floor conveyor to provide the required doses of specific macronutrient expressed in kg/ha.

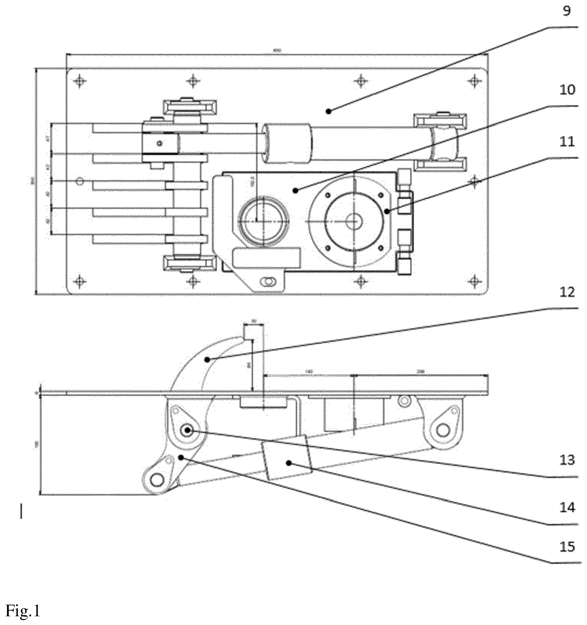

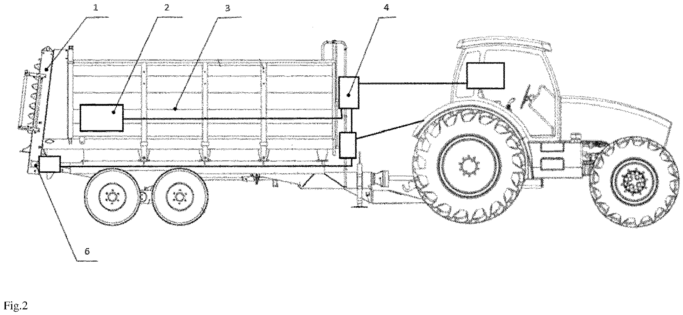

The object according to the invention is explained in the examples of embodiment in the drawing, in which fig. 1 shows a device for the precise application of solid organic fertilisers especially in manure spreaders, fig. 2 shows the components of the device located on the spreader assembly and the tractor working with this spreader.

### Example of the embodiment of the invention

The device for the precise application of solid organic fertilisers especially in manure spreaders consists of a near-infrared spectrometer 2 together with a measuring probe installed in the loading box 3 of a manure spreader equipped with a floor conveyor 6 and a spreading adapter 1. The measuring probe 9 of the near-infrared spectrometer 2 is mounted in the spectrometer probe holder 11 on a mounting plate 9 installed on the side wall of the manure spreader loading box 3 in that part of the manure spreader loading box 3 which is directly adjacent to the spreading adapter 1, and is positioned in the lower half of the loading box 3 of the manure spreader above the floor conveyor 6, while immediately in front of the measuring probe there is a set of retaining tines 12 set coaxially on a rotary axis 13, connected to a lever 15 set in motion by a hydraulic cylinder 14.

The mounting plate 9 is installed on the side wall of the loading box 3 of the manure spreader, and the measuring probe installed thereon is advantageously not more than one metre away from the spreading adapter 1. It is advantageous if the measuring probe on the mounting plate 9 is installed at a height of not more than 300 mm above the floor conveyor 6 and not less than 100 mm above the floor conveyor 6.

It is advantageous if there is an inspection window 10 on the side wall of the manure spreader loading box 3 and the mounting plate 9 at the mounting point of the probe holder of the spectrometer 2.

The method for the precise application of solid organic fertilisers especially in manure spreaders consists of the steps of: mapping the vegetation of the field, placing the solid organic fertiliser in the loading box 3 of the manure spreader, testing the composition of the organic fertiliser using the measuring probe of the near-infrared spectrometer 2, sending the results via an interface (USB, Ethernet, CAN Bus) to the controller 4 managing the operation of the manure spreader, adjusting the speed of the fertiliser spreading by spreading adapter 1 to the results obtained estimating the fertiliser's NPK macronutrient abundance using spectrometer 2 by varying the speed of the floor conveyor 6, whereby, prior to the start of the test of the organic fertiliser composition by means of the measuring probe of the near-infrared spectrometer 2, the extending piston rod of the hydraulic cylinder 14 causes the fertiliser sample retaining tines 12 to slide into the loading box 3 of the manure spreader and simultaneously to move them to the front of the box 3, there is a counter-direction of this movement in relation to the movement of the fertiliser inside the loading box 3 and the fertiliser is compressed and pressed against the wall of the loading box 3, in which the measuring probe is placed and the composition of the fertiliser is estimated by means of the spectrometer on the basis of the absorbance spectrum acquired with the measuring probe, after the measurement the piston rod of the cylinder 14 retracts into the cylinder releasing the retained fertiliser sample, the retaining tines 12 retract into the wall of the loading box 3 allowing further free flow of material until the next measurement is started.

## Claims

1. Device for the precise application of solid organic fertilisers in particular in manure spreaders, consisting of a near-infrared spectrometer (2) with a measuring probe installed in the loading box (3) of a manure spreader equipped with a floor conveyor (6) and a spreading adapter (1), **characterised in that** the measuring probe of the near-infrared spectrometer (2) is mounted in the probe holder (11) of the spectrometer on a mounting plate (9) installed on the side wall of the loading box (3) of the manure spreader **in that** part of the loading box (3) of the manure spreader, which is directly adjacent to the spreading adapter (1) and is spaced from it not more than 100 cm and is positioned in the lower half of the manure spreader loading box (3) above the floor conveyor (6) but not more than 30 cm from the floor conveyor (6), while directly in front of the measuring probe there is a set of retaining tines (12) set coaxially on a rotary axis (13) connected to a lever (15) set in motion by a hydraulic cylinder (14).

2. The device according to claim 1, **characterised in that** the mounting plate (9) is installed on the side wall of the loading box (3) of the manure spreader and the measuring probe installed thereon is located not more than one metre away from the spreading adapter (1).

3. Device according to claim 1 or 2, **characterised in that** the measuring probe on the mounting plate (9) is installed at a height of not more than 300 mm above the floor conveyor (6).

4. Device according to claim 1 or 2, **characterised in that** the measuring probe on the mounting plate (9) is installed at a height of not less than 100 mm above the floor conveyor (6).

5. Device according to claim 1 or 2 or 3, **characterised in that** an inspection window (10) is provided on the side wall of the loading box (3) of the manure spreader and on the mounting plate (9) at the mounting location of the holder (11) of the spectrometer probe (2).

6. A method for the precise application of solid organic fertilizers especially in manure spreaders consisting of the steps of: mapping the vegetation of the field, placing the solid organic fertiliser in the loading box (3) of the manure spreader, testing the composition of the organic fertiliser using the measuring probe of the near-infrared spectrometer (2), sending the results via an interface (USB, Ethernet, CAN Bus) to the controller (4) managing the operation of the manure spreader, adjusting the fertiliser spreading speed by the spreading adapter (1) to the obtained results of estimation of the fertiliser NPK macronutrient abundance by means of the spectrometer (2) by varying the speed of the floor conveyor (6), **characterised in that**, prior to starting the testing of the composition of the organic fertiliser using the measuring probe of the near-infrared spectrometer (2), the extending piston rod of the hydraulic cylinder (14) causes the fertiliser sample retaining tines (12) to slide into the loading box (3) of the manure spreader and simultaneously to move them to the front of the box (3), there is a counter-direction of this movement in relation to the movement of the fertiliser inside the loading box (3) and the fertiliser is compressed and pressed against the wall of the loading box (3) in which the measuring probe is placed and the composition of the fertiliser is estimated with the spectrometer on the basis of the absorbance spectrum acquired with the measuring probe, once the measurement has been taken, the piston rod of the cylinder (14) retracts into the cylinder releasing the retained fertiliser sample, the retaining tines (12) retract into the wall of the loading box (3) allowing the material to continue to flow freely until the next measurement is taken.
